# EUROPEAN PATENT APPLICATION

(11) **EP 1 298 587 A2**
(43) Date of publication of application: **02.04.2003**
(21) Application number: 02021617.2
(22) Date of filing: 27.09.2002
(51) Int. Cl.: G06T 5/00

(54) **Body image enhancement**

(30) Priority: 01.10.2001 US 325559 P; 21.12.2001 US 24480
(71) Applicant: L'OREAL, 75008 Paris (FR)
(72) Inventor: Rubinstenn, Gilles, 75005 Paris (FR); Pruche, Francis, 60300 Senlis (FR)
(74) Representative: Tanty, François

(57) **Abstract**

Systems, methods, and apparatus consistent with the present invention may be used to enable a subject to alter an image of the subject's body based on the subject's self-evaluation of the actual condition of the subject's own body. The subject may be prompted to capture, using an image capture device, an initial body image. The subject may also be prompted to self-evaluate the actual condition of the subject's own body. Based on the subject's response to the self-evaluation prompt, the initial body image may be altered to reflect the self-evaluation of the subject.

## Description

The present invention generally relates to interactive computer systems, and more specifically to systems, methods, and apparatus for constructing an image reflective of a subject's perception of reality. In one example, a captured image of a subject may be altered to more accurately reflect a subject's perception of an external body condition.

Although the present invention, in its broadest sense, is not limited to the cosmetic market, cosmetics will be used herein to convey some of the aspects associated with the invention.

It is recognized that people are not always flattered, or even satisfied, with images of themselves. Often, this negative reaction can be attributed to a person's perception of reality (how they look) conflicting with what the image (e.g., photograph) portrays. For example, a person may visualize herself as having a golden brown skin tone, but perceive her skin tone in an image to be pale. This predicament may be the result of inaccuracies in the captured image due to, for example, environmental conditions such as poor lighting. People may also have difficulties with perceiving or associating multi-dimensional images in a two-dimensional medium.

Devices such as web cams and associated devices often exacerbate the dilemma: low-resolution web cams and/or monitors, poor lighting, insufficient software, and other adverse factors may cause a person's image to appear inaccurate or unflattering. Even if the equipment functions to provide an image which is substantially accurate, a person's skewed perception of reality may cause the person to be uncomfortable with what the image portrays. Nevertheless, regardless of the reason, people are not always comfortable viewing images of themselves.

This lack of comfort or negativity may be detrimental to certain products and services in which captured images play an integral role. One example is an on-line computer-based service that uses captured facial images to provide cosmetic advice. If consumers using such a system have negative reactions to their images, they will undoubtedly lose interest or become discouraged from using the service.

Accordingly, it may be beneficial to provide consumers with the ability to construct or alter captured images of themselves in accordance with their perceptions of reality.

Systems, methods, and apparatus consistent with principles of the present invention, optionally may address one or more of the above problems and/or other problems by providing methods for altering body images based on a subject's self-evaluation of her own body.

One aspect of the invention may involve constructing an image of a subject's external body condition. Methods of the present invention may be provided for prompting a subject to capture, using an image capture device, at least one initial image of a subject's body. A subject may be prompted to perform a self-evaluation of an actual condition on the subject's body, such as wrinkles around the subject's eyes, for example. The present invention may enable the subject to respond to the prompt. Methods may be provided for altering the initial image to reflect the subject's self-evaluation of the actual body condition. For example, the subject may be able to increase the intensity and/or number of wrinkles around the eyes by activating and/or moving one or more control elements.

One aspect of the present invention may involve prompting the subject to self-evaluate the actual color and/or texture of an external body condition. Methods may be provided for allowing the subject to respond to the prompt. An enhanced image may be generated, wherein the enhanced image is intended to more accurately portray the external body condition. This enhanced image may be presented to the subject, and the subject may be prompted to indicate the accuracy of the enhanced image. Methods of the present invention may provide for altering the enhanced image when the subject indicates that the enhanced image is inaccurate.

Another aspect of the present invention may involve prompting a subject to input a facial image into a computer. For example, capturing an image with a web cam and inputting the image into a computer. Consistent with principles of the present invention, the facial image may include at least one bias element (e.g., pale skin tone), which causes the subject to perceive that the image inaccurately portrays reality. Methods of the invention may include enabling an identification of the bias element, and allowing the subject to participate in selecting a new visual element to replace the identified bias element. An altered facial image may be constructed by replacing the identified bias element with the new visual element, and the altered image may be displayed to the subject. In alternative embodiments, bias elements may be automatically detected, and optionally replaced, before the image is presented to the subject.

In another aspect, the present application involves a method of enabling color-calibrating of a self-image for use in simulating a beauty product use. The method includes prompting a subject to capture, using an image capture device, an image of a body region of the subject; enabling the display of the captured image to the subject on a display device; prompting the subject to compare a color of the displayed image with an actual color of the subject; enabling the subject to calibrate the color of the image when the subject perceives a difference between the displayed image and the actual skin color; and enabling the subject to simulate use of at least one beauty product on the color calibrated image.

In a further aspect, there is a method of color calibrating including capturing, using an image capture device, an image of a body region; viewing display of the captured image on a display device; comparing a color of the displayed image with an actual color of the body region; calibrating the color of the image when a difference is perceived between the displayed image and the actual color of the body region; and causing a simulation of use of at least one beauty product on the color calibrated image.

In another aspect, the present application involves a method of constructing a facial image, the method comprising:
prompting a subject to cause input into a computer of a facial image of the subject, wherein the facial image includes at least one bias element that may tend to cause the subject to perceive that the facial image may inaccurately portray reality;
enabling an identification in the facial image of the at least one bias element;
enabling the subject to participate in selecting a new visual element to replace the at least one identified bias element;
enabling the construction of an altered image from the facial image by replacing the at least identified one bias element with the new visual element; and
enabling the altered image to be displayed to the subject.

The prompting may comprise prompting the subject to capture the facial image using an image capture device and to input the facial image to the computer.

Enabling an identification may comprise enabling the subject to identify the at least one bias element, and/or may comprise enabling the subject to be informed about presence of the at least one bias element.

The at least one bias element may be chosen from at least one of skin pigmentation, skin texture, skin sheen, skin tone, skin mattiness, skin wrinkles, and skin lines.

Enabling the subject to participate in selecting the new visual element may comprise causing at least one control element to be displayed on a display device and enabling activation of the control element to viewing differing visual elements and/or causing a plurality of selectable condition representations to be displayed on the display device, and enabling the subject to select at least one of the condition representations.

The method may further comprise causing display, on a display device, of a representation of the facial image having fuzzy distortion.

The displayed facial image may be altered based on the selection of the new visual element, to remove at least a portion of the fuzzy distortion.

Each selection of a new visual element may cause further removal of the fuzzy distortion.

The displayed image may be altered so that a representation of the new visual element is substituted in place of said at least a portion of the fuzzy distortion.

In another aspect, the present application involves a method of constructing a body image, the method comprising:
causing presentation of at least one initial body image of the subject;
causing presentation of at least one prompt prompting the subject to self-evaluate an actual condition of the subject's own body;
enabling the subject to respond to the at least one prompt; and
enabling the initial body image to be altered based on the subject's response to the at least one prompt, to thereby reflect in the altered image the self-evaluation of the subject.

In another aspect, the present application involves a method of enabling color-calibrating of a self-image for use in simulating a beauty product use, the method comprising:
prompting a subject to capture, using an image capture device, an image of a body region of the subject;
enabling the display of the captured image to the subject on a display device;
prompting the subject to compare a color of the displayed image with an actual color of the subject;
enabling the subject to calibrate the color of the image when the subject perceives a difference between the displayed image and the actual skin color; and
enabling the subject to simulate use of at least one beauty product on the color calibrated image.

The method may further comprise causing the image to be processed in a manner enabling simulated use of the at least one beauty product on predetermined portions of the image.

The image capture device may be chosen from a digital camera and a scanner.

Prompting the subject to compare the color may comprise prompting the subject to place the body region adjacent to the display device and to visually perceive whether the color of the displayed image and the actual color of the body region differ.

Enabling the subject to calibrate color may comprise enabling the display of a plurality of colors, enabling the subject to select one of the displayed colors closest to the actual color of the subject's body region, and enabling alteration of the displayed image to include the selected color.

Enabling the subject to simulate use of at least one beauty product may comprise enabling the subject to select the at least one beauty product from a plurality of beauty products and causing a simulation of use of the selected beauty product to appear on a region of the color calibrated image.

Enabling the subject to calibrate may enable the subject to calibrate the displayed image to simulate at least one of an actual skin tone and an actual hair color.

The method may further comprise a method of color-calibrating an image for use in simulating a beauty product use, the method comprising:
capturing, using an image capture device, an image of a body region;
viewing display of the captured image on a display device;
comparing a color of the displayed image with an actual color of the body region;
calibrating the color of the image when a difference is perceived between the displayed image and the actual color of the body region; and
causing a simulation of use of at least one beauty product on the color calibrated image.

The image may be processed in a manner enabling simulated use of the at least one beauty product on predetermined portions of the image.

The image capture device may be chosen from a digital camera and a scanner.

Comparing the color may comprise placing the body region adjacent to the display device and visually perceiving whether the color of the displayed image and the actual color of the body region differ.

Calibrating the color may comprise viewing display of a plurality of colors, selecting one of the displayed colors closest to the actual color of the body region, and causing alteration of the displayed image to include the selected color.

Causing simulated use may comprise selecting the at least one beauty product from a plurality of beauty products and causing a simulation of the selected beauty product to appear on a region of the color calibrated image.

The calibrating may comprise calibrating the displayed image to simulate at least one of an actual skin tone and an actual hair color.

It is to be understood that both the foregoing and the following descriptions are exemplary and explanatory only and are not intended to limit the claimed invention in any manner whatsoever.

The accompanying drawings, which are incorporated in and constitute a part of this specification exemplify certain aspects of the present invention and together with the description, serve to explain some principles of the invention.
Figure 1 depicts an exemplary screen shot consistent with the present invention;
Figure 2 is a flowchart consistent with methods of the present invention;
Figures 3a and 3b depict exemplary screen shots consistent with the present invention;
Figures 4, 5, 6a, 6b, 7a, and 7b are other exemplary screen shots consistent with principles of the present invention;
Figure 8 is an exemplary block diagram of a system in which the present invention may be practiced;
Figure 9 is a detailed block diagram representative of an access system depicted in the system of Figure 8;
Figure 10 is a detailed block diagram representative of a server system illustrated in the system of Figure 8;
Figure 11 is detailed a flowchart consistent with one exemplary embodiment of the present invention;
Figure 12A is a flowchart of an exemplary calibration method consistent with the present invention;
Figure 12B is a flowchart of the exemplary calibration method from the user's perspective, consistent with the present invention; and
Figure 13 is an exemplary user interface for use with the exemplary color calibration method, consistent with the present invention.

In the following description of exemplary embodiments, reference will be made to the accompanying drawings in which like numerals represent the same or like elements.

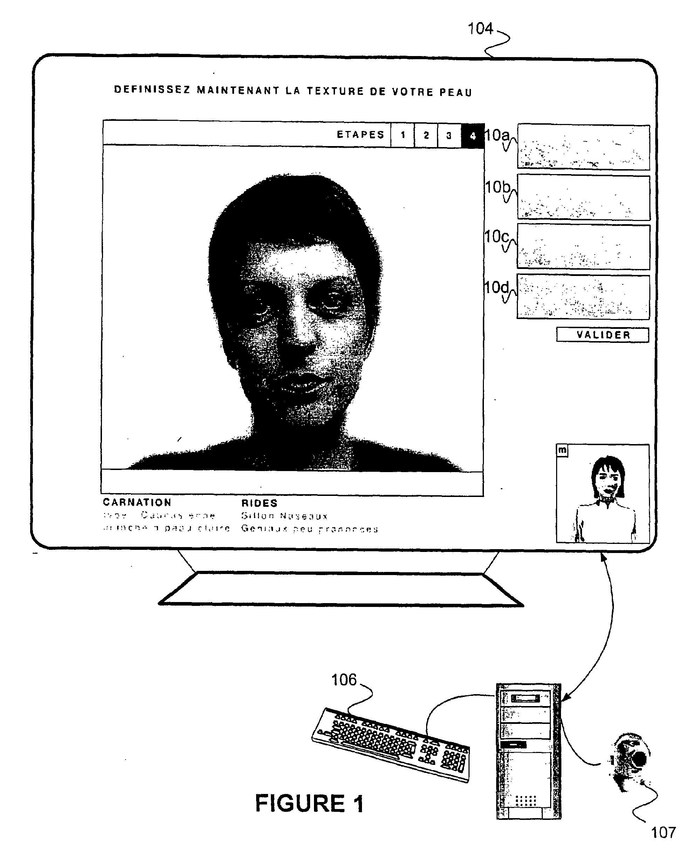

Consistent with exemplary embodiments of the present invention, methods may be provided for allowing subjects to alter a body image to more accurately reflect reality. One embodiment of the present invention is illustrated, by way of example, in the screen shot of Figure 1. As illustrated in Figure 1, the present invention may involve displaying, via computer monitor 104, a facial image of a subject captured via camera 107. In addition, a plurality of selectable condition representations (e.g., skin textures 10a, 10b, 10c, and 10d) may also be displayed. The screen shot display of Figure 1 may be configured to provide a prompt suggesting that the subject evaluate her actual skin texture and this display may be further configured to allow the subject to modify the image to more appropriately reflect her actual skin. For example, the subject may be able to select, using keyboard 106, mouse, trackball, interactive voice response system (IVR), voice recognition or any other user interface that allows a subject to input data, the skin texture representation 10a, 10b, 10c, 10d, which most closely resembles her actual skin. With each selection, the image may be modified to include the selected representation and then displayed to the user for further evaluation.

The foregoing discussion is intended to introduce and provide initial clarity for some of the aspects associated with the present invention by referring to the exemplary embodiment depicted in Figure 1. Further details of this embodiment as well as additional aspects and embodiments of the present invention will be described in the following discussion. However, it is to be understood that other alternative embodiments may be utilized and that structural and method changes may be made without departing from the scope of present invention. The foregoing and following discussion are, therefore, not to be construed in a limiting sense.

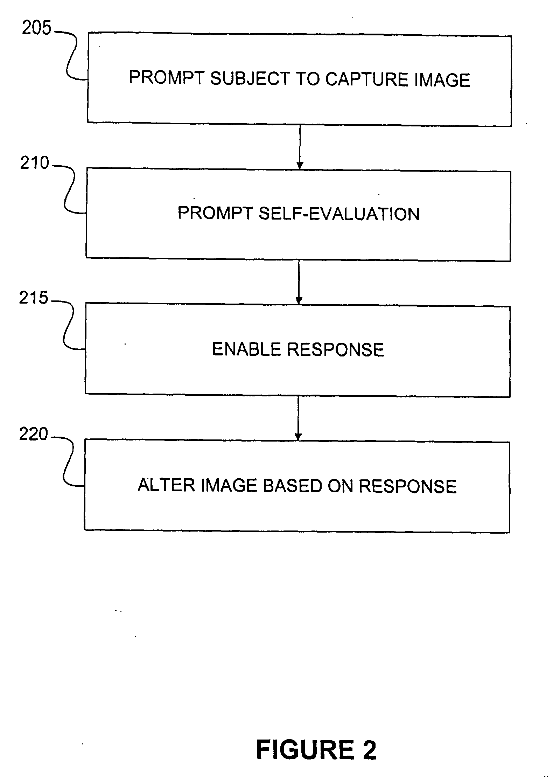

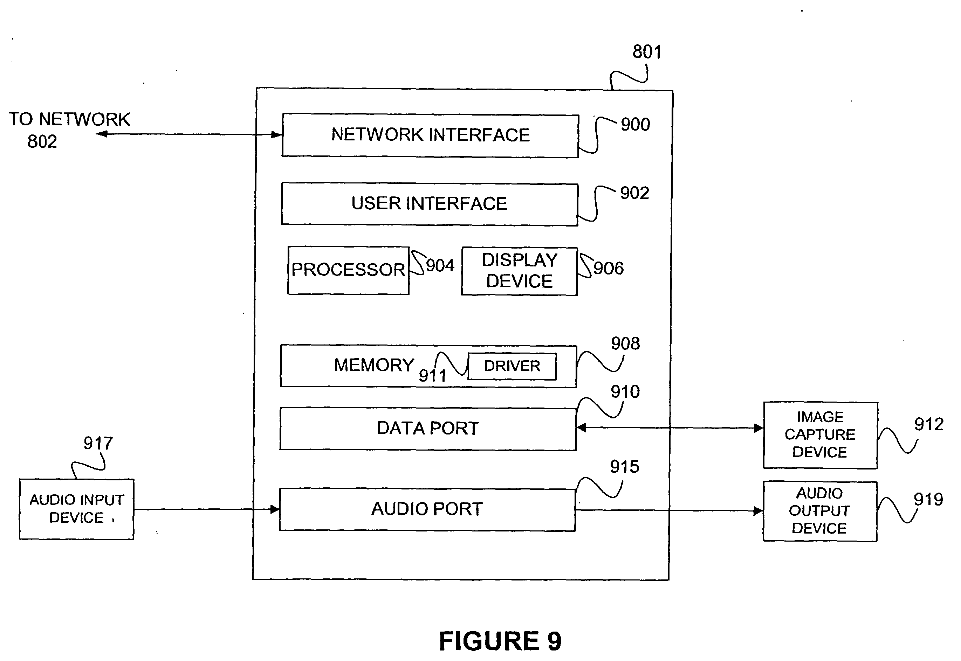

A method consistent with the present invention, may include prompting a subject to capture, using an image capture device (e.g., camera 107), at least one initial body image of the subject. This is indicated by step 205 in the flowchart of Figure 2. The details of the image capture device will be discussed later in connection with Figure 9. A "body image" may include, but is not limited to, a two or three dimensional likeness of all (or one or more portions of) a subject's face, or other portions of a subject's body. As used herein, "prompting" refers to encouraging a subject to perform an action or causing the subject to receive such encouragement. For example, prompting could be accomplished by transmitting software and/or computer readable data from a location remote from the location of a device intended to be used by the subject (e.g., via a network, via a portable storage media, and/or via a hard copy). Prompting may also include publishing, advertising, distributing, and/or conveying information in any other way that encourages capture of the subject's body image.

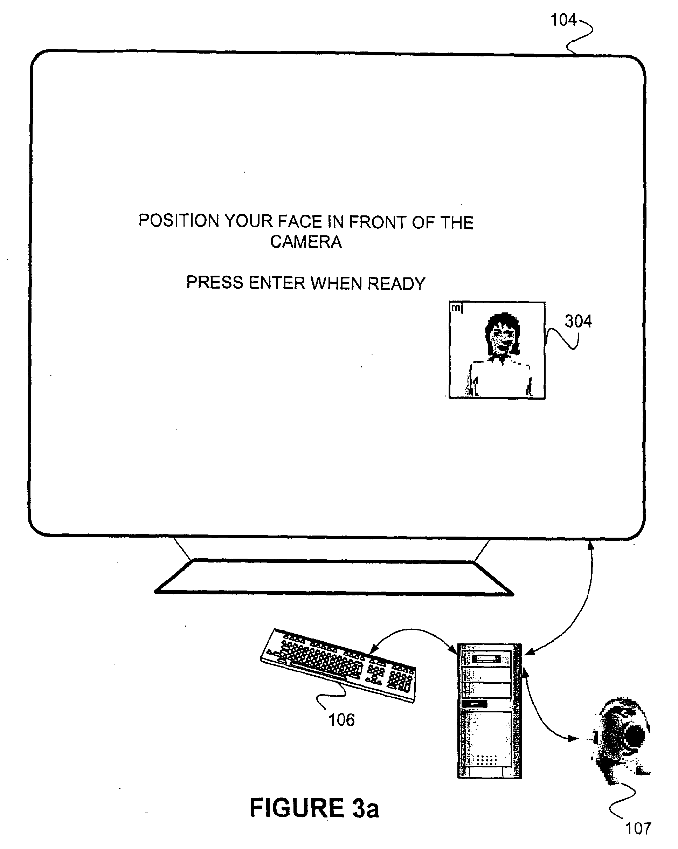

In the exemplary embodiment depicted in Figures 3a and 3b, prompting the subject may include instructing the subject to use camera 107. As illustrated, this may be accomplished by causing textual messages to be displayed via a display device (e.g. computer monitor 104). However, in alternative embodiments, prompting may involve causing audible cueing of the subject via an audio output device, such as a speaker, or graphical cueing of the subject with an image or icon. Prompting may also involve causing a human image 304 to audibly and visually present instructions to the subject in a manner consistent with a live human being.

As illustrated in the example of Figures 3a and 3b, the subject could be presented with an initial prompt, shown in Figure 3a, prompting the subject to position her face adjacent to camera 107, and then a further prompt, shown in Figure 3b, prompting the subject to turn her head. Prompting the subject to orient herself in this fashion may enable one or more images to be captured from a variety of different perspectives, while the subject is in the process of turning her head, for example. In certain examples consistent with the invention, a plurality of images from different perspectives may be used to construct a three-dimensional image of the subject's face or a portion of the subject's face, and such a three-dimensional image might be the initial body image used in a beauty analysis.

In a broader sense, only a single image (e.g., either two-dimensional or three-dimensional) may be captured and this single image may be used as the initial body image in a beauty analysis. In one alternative embodiment, one or more two dimensional images may be captured and applied on a three-dimensional representation (e.g., model) to generate an initial three-dimensional image that may be used in a beauty analysis. One example such a three-dimensional image generation is discussed below.

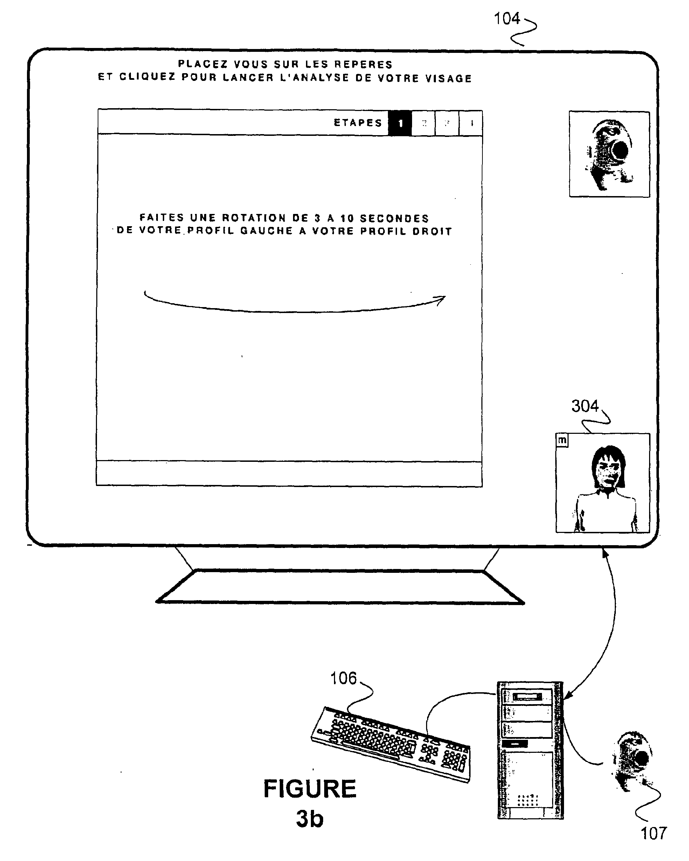

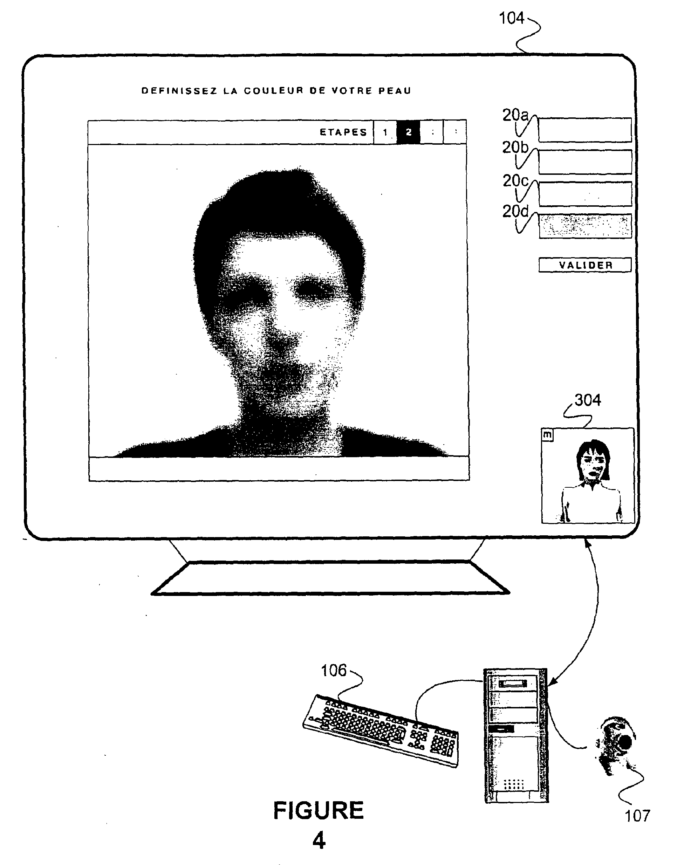

Once an initial body image is captured, the image may be stored and presented to the subject. Images may be stored in memory, storage media (e.g., floppy disks), and/or in a database, the details of which will be discussed later in connection with Figures 9, 10, and 11. In exemplary embodiments, presentation of the initial image may occur on computer monitor 104, as illustrated in the figures, or on any other type of display device. In one embodiment, the initial image presented on the display device may be displayed with fuzzy distortion (e.g., blurred), as illustrated in Figure 4. This may be accomplished by an image processing mechanism. The fuzzy distortion may be used to prevent the subject from having an initial negative reaction to the image. For example, the fuzzy distortion may be used to block certain features of the subject's face, which the subject is not readily comfortable with viewing. The distortion might also block image imperfections possibly associated with the technique of capturing the image.

In accordance with principles of the present invention, captured images may contain bias elements. As used herein, the term "bias element" refers to any visual element or aspect of an image, which may tend to cause the subject to perceive that the image inaccurately portrays reality. In this context, "reality" refers to either what actually exists, or the subject's perception of what actually exists. Bias elements may be present in the images as a result of a variety of differing causes, such as environmental conditions, equipment deficiencies, and/or user error. For example, bias elements may include shadows or color misrepresentations. Bias elements may affect any portion of the image. For instance, bias elements may include, but are not limited to, skin pigmentation, skin texture, skin sheen, shin tone, skin mattiness, skin wrinkles, and skin lines. In one alternative embodiment, bias elements may be purposefully injected and/or removed by image processing. For example, the initial image may be presented with a pre-determined neutral skin tone and/or texture, which may then modified by the subject to more accurately reflect reality. In addition (or in the alternative), the initial images might lack any wrinkles, and then wrinkles could be added by the subject according to the subject's own perception of her wrinkles.

Consistent with principles of the present invention, methods may be provided for causing presentation of at least one prompt for prompting the subject (or someone acting on the subject's behalf) to self-evaluate an actual condition of the subject's own body, as indicated in step 210 of Figure 2. As used herein, an "actual condition" refers to at least one of skin pigmentation, skin texture, skin sheen, skin tone, skin mattiness, skin lines, skin wrinkles, distribution of wrinkles, intensity of wrinkles, intensity of pores, depth of pores, color tone, color homogeneity, spots, freckles, shininess, oiliness, roughness, distribution of hair (e.g., scalp hair or facial hair), thickness of hair, length of hair, density of hair, or any other skin condition and/or hair condition. The actual condition may reside on any region of the subject's body. For example, in one embodiment, the actual condition may be located on regions of the subject's face including one or more of the eyes, forehead, cheeks, lips, brow, nose, and chin. Prompting may involve techniques similar to those discussed above in connection with step 205.

As illustrated in Figure 4, a message may be displayed cueing the subject to evaluate her skin tone. In addition (or in the alternative) to instructing the user to perform a self-evaluation, the prompting could include causing one or more queries to be presented to the subject, wherein the queries relate to the self-evaluation. For example: "Does the displayed image appear to accurately reflect your actual skin tone?"

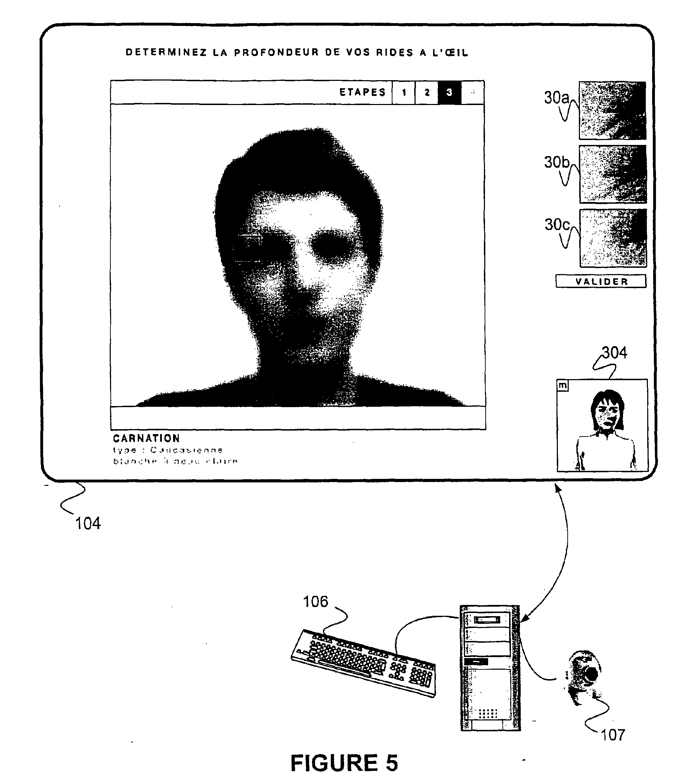

Another example may involve accentuating or extracting a portion of the initial image for evaluation. Such a technique may be used for the example of Figure 5, which relates to wrinkles in the regions around the eyes.

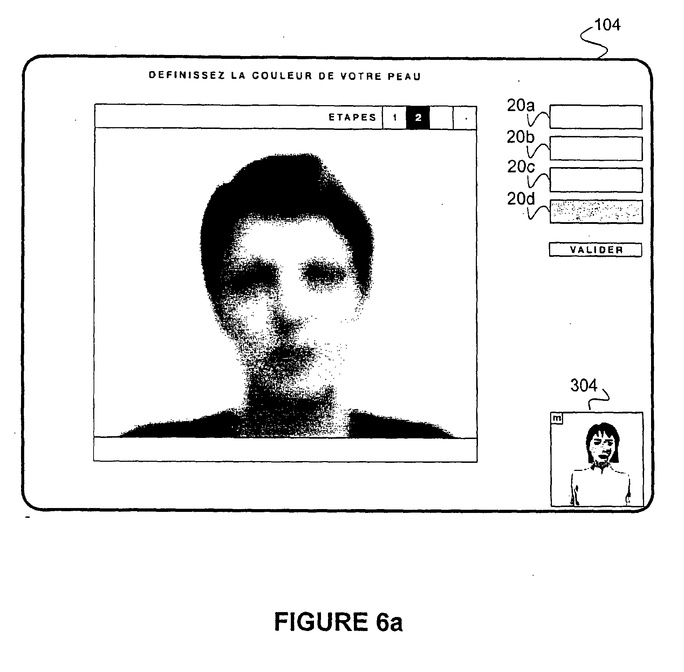

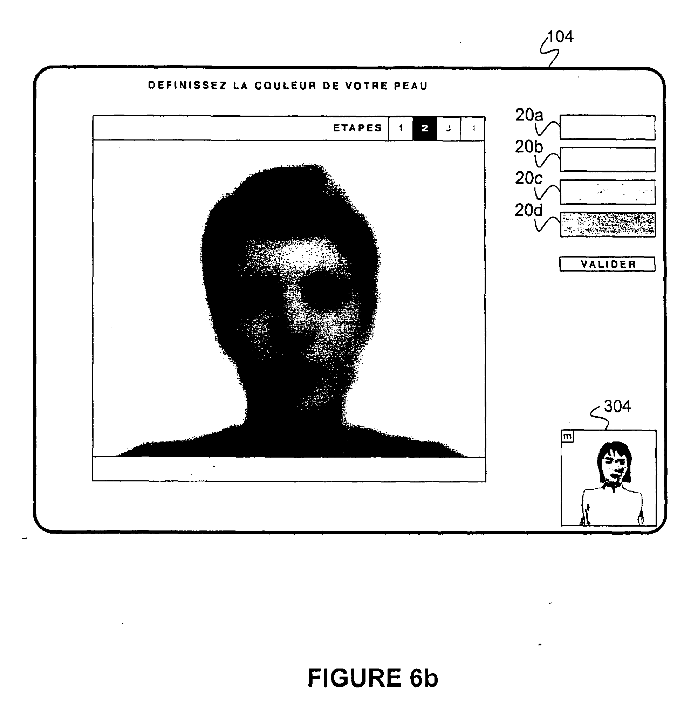

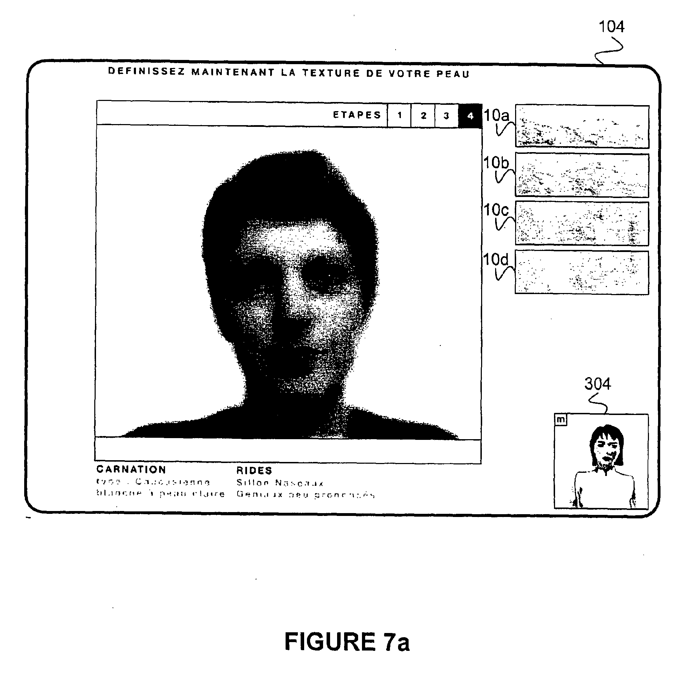

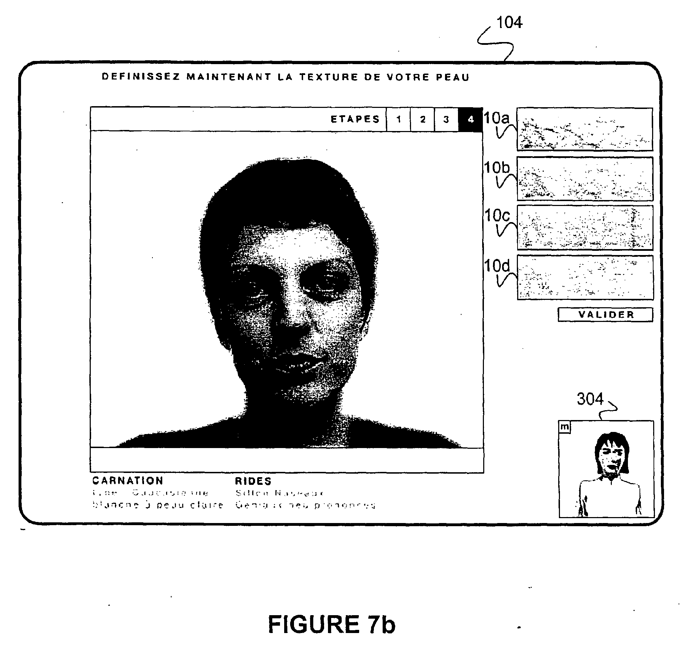

Prompting may further involve presenting the subject with a plurality of selectable condition representations from which to choose. For example, Figures 4, 6a, and 6b show an example of selectable skin tone images 20a, 20b, 20c, 20d; Figure 5 shows an example of selectable eye region wrinkles 30a, 30b, 30c; and Figures 1, 7a, and 7b show an example of selectable skin texture images 10a, 10b, 10c, 10d. In the alternative (or in addition) to displaying a plurality of representations simultaneously, the representations may be displayed via a one-by-one fashion, such as in a slide show presentation and/or a movie presentation.

Performing a self-evaluation may involve comparing all or a portion of the initial body image with the subject's actual body in order to determine if the initial image accurately depicts reality. For example, the subject may compare the intensity of wrinkles around her eyes with what the initial body image depicts. A self-evaluation may also entail evaluating bias elements. A further example of a self-evaluation might involve comparing the above-mentioned representations with the subject's body

As illustrated in step 215 of Figure 2, a method of the present invention may include enabling the subject to respond to the prompt. In a broad sense, responding may involve indicating whether the initial image accurately represents the subject's actual body. Responding may include, but is not limited to, selecting one or more of the above-mentioned selectable condition representations, choosing an item from a multiple choice list, checking a box, typing in a textual response in a text field, audibly speaking into an audio capture device (e.g., a microphone), or any other input of the subject. In one embodiment, responding may further include allowing the subject to manipulate a control element, which incrementally alters the initial image. For example, a sliding scale may be graphically presented, which (when moved by the subject) increases and decreases the intensity of wrinkles on a portion of the displayed facial image. Alternatively (or in addition), a plurality of control elements could be displayed, wherein activation of one control element increases displayed wrinkles and activation of another control element decreases displayed wrinkles.

In another sense, responding may involve enabling the subject to participate in selecting a new visual element to replace one or more bias elements. Accordingly, methods may be provided for identifying these bias elements. This may involve highlighting, accentuating, or extracting the bias element portions of the image or enabling the subject to do so. This may also involve focusing the subject's attention on the elements. For example, audible and/or visual cues may be used to articulate the existence of bias elements.

Consistent with exemplary embodiments of the present invention, methods may be provided for altering the initial image of the subject based on the subject's response to the prompt, to thereby reflect the self-evaluation performed by the subject. This is indicated by step 220 of Figure 2. In one exemplary embodiment, altering may be performed by conventional image processing, such as morphing. Altering may involve modifying the initial captured body image to more accurately reflect the subject's perception of reality. With regard to the examples shown in Figures 1, 4, 5, 6a, 6b, 7a, and 7b, the body image could be altered to include the condition representation selected by the subject. For example, Figures 6a and 6b respectively illustrate a body image before and after one of the skin tone representations 20a, 20b, 20c, 20d is selected. The skin tone on the displayed facial image of Figure 6b is darker than that of Figure 6a because the subject has selected a deeper tone, for example.

In exemplary embodiments, altering may involve incrementally decreasing the fuzzy distortion of the displayed body image as the subject makes selections and/or substituting the selected representation(s) in place of the distortion. For example, Figures 7a and 7b respectively illustrate a body image before and after one of the skin texture representations 10a, 10b, 10c, 10d, has been selected. As illustrated by comparing Figure 7b to Figure 7a, the facial image may become more clear after a skin texture is selected.

In other embodiments, certain image alterations may be performed automatically by image processing, without subject input. For example, certain bias elements may be automatically detected, and the image may be altered prior to display.

In yet another embodiment, the altered body images may be further altered to depict a time-lapse projection. For example, the subject may be able to view further altered images, reflecting the use of beauty products, or the affects of following (or not following) a particular beauty regimen. A time-lapse projection may include, but is not limited to, displaying images simultaneously, in sequence, in a slide show format, and/or in a movie format.

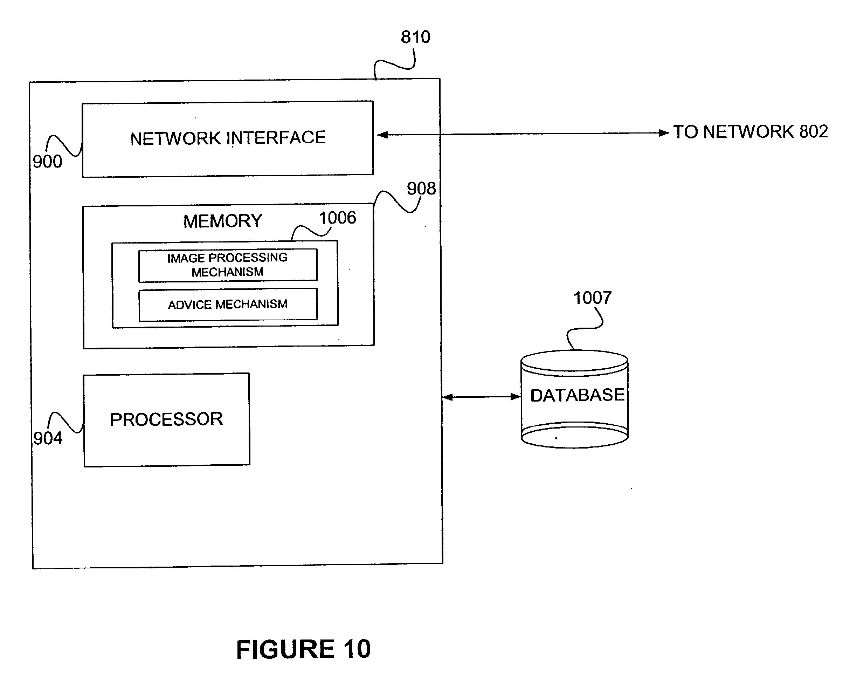

In accordance with principles of the present invention, methods may be provided for providing beauty advice to the subject. Advice may include, but is not limited to, beauty product recommendations (e.g., recommendations for cosmetic substances and/or cosmetic services to treat conditions the subject is prompted to evaluate), remedial measures, and preventative measures. Accordingly, the above-mentioned time-lapse projection may depict the results of following the beauty advice. Consistent with methods of the present invention, an advice mechanism may be provided for supplying this beauty information. The details of such a mechanism will be discussed later in connection with Figure 10.

The methods might also include enabling the subject to simulate use of one or more beauty products (e.g., cosmetic products) on the altered image. The subject may be given the option of selecting the beauty product from a plurality of differing beauty products. The beauty products may be products for treating conditions and/or makeup products. The methods might also involve causing the subject to be presented with information enabling the subject to purchase one or more products and/or receiving such a purchase order.

Software for processing of images for use in simulated cosmetic products is also available from EZ-Face of Israel.

The method might also involve enabling the subject to store the altered image on any type of data storage device or media so that the image may be recalled by the subject.

Further examples of the method might also involve prompting the subject to provide an indication of whether the altered image is accurate and enabling the subject to respond. For example, such prompting and response to the prompting could be similar to the prompting and response described in connection with other aspects of the method.

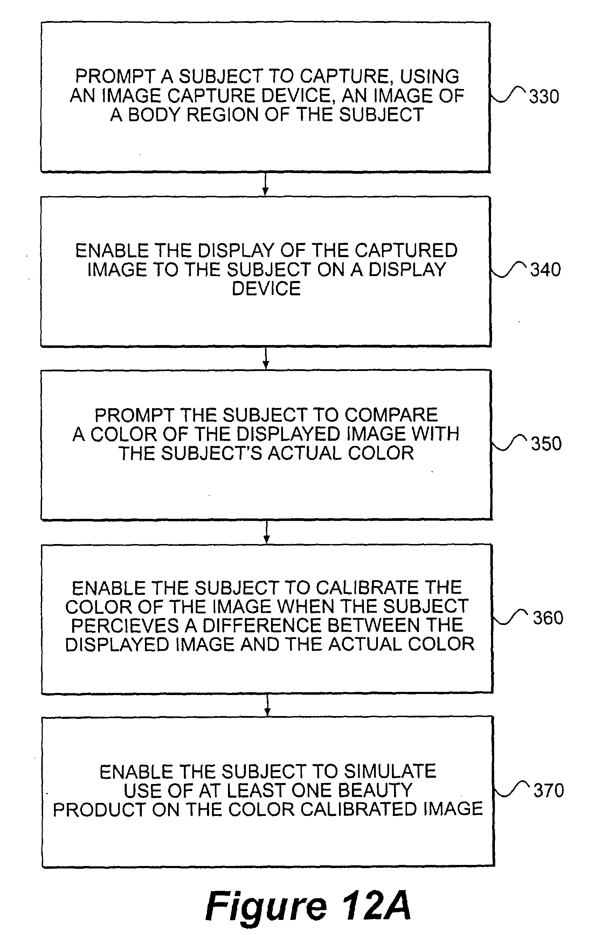

Figure 12A is a flow chart of an exemplary calibration method consistent with the invention. As explained in more detail below, the method may involve prompting a subject to capture, using an image capture device, an image of a body region of the subject (330); enabling the display of the captured image to the subject on a display device (340); prompting the subject to compare a color of the displayed image with the subject's body actual color (350); enabling the subject to calibrate the color of the image when the subject perceives a difference between the displayed image and the actual color (360); and enabling the subject to simulate use of at least one beauty product on the color-calibrated image (370).

As used herein the term "color-calibrating" includes, but is not limited to, matching an actual color of the subject's skin with, for example, a color of the subject's skin that is displayed on a display device.

Prompting the user to capture a body region image (330) may be through a website or may occur by conveying a program to a machine accessed by the user. Prompting may also include one or more of providing instructions on how to go about capturing an image, providing a driver for an image capture device, providing image capture software, or providing access to a network site for facilitating image capture. Examples of image capture devices consistent with the invention may include, but are not limited to, web cams, digital cameras, analog cameras, scanners, and any other mechanism for capturing a visual representation of a body image.

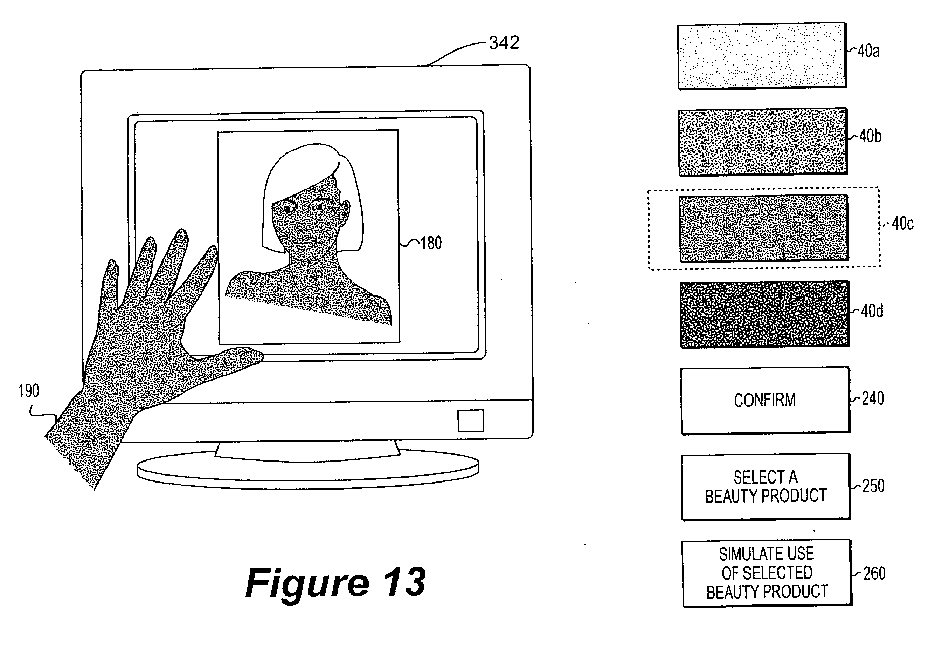

The method of Figure 12A may further include enabling the display of the capture image to the user on a display device (340). Figure 13 shows an example of a captured image 180 being displayed on a display device **342.** As used herein, the term "enabling display" is not limited to the direct act of displaying. It also includes indirect acts such as providing the user with access to software for causing the display to appear.

Once the image is displayed, the method may include prompting the subject to compare a color of the displayed image with the subject's actual color (350). For example, the subject may be prompted to compare the color of a displayed body region to the actual skin color of that body region. The subject may be prompted using text commands, voice commands, or through any other instructions eliciting a comparison.

Figure 13 illustrates how a subject might compare the color of the displayed image 180 with the actual color of her hand 190 by placing her hand adjacent to the display device. The prompting of step 350 may encourage the subject to make the comparison by providing the subject with directions or instructions to do so. For example, when the skin color (e.g., tone) on the subject's hand differs from the actual skin color of the body region, the subject may be prompted to make a more precise comparison (e.g., in Fig. 13, comparing the captured facial image with the subject's actual facial skin color rather than the skin color of the subject's hand).

Enabling the user to calibrate color may include enabling the display of a plurality of colors, enabling the subject to select one of the displayed colors closest to the actual color of the subject's body region, and enabling alteration of the displayed image to include the selected color. These actions may occur directly or indirectly. For example, the subject may be presented with a plurality of controls 40a, 40b, 40c, and 40d, each corresponding to a differing color, from which a particular color (e.g., 40c) may be selected (Figure 13). The subject may be presented with a confirm button 240 to alter the displayed image to include the selected color, for example. As an alternative to exemplary displayed color controls 40a, 40b, 40c and 40d, a sliding continuum may be provided or the subject may be provided with some other control feature for making a positive color identification.

In a manner similar to that illustrated in Figure 13, a subject may be enabled to select an actual hair color and/or an actual eye color.

Consistent with the invention, the user may be enabled to simulate use of at least one beauty product on the color-calibrated image (370). Such enabling may occur through the provision of the beauty product simulation control 260 (Figure 13). Such control button may be presented to the subject, for example, after at least one beauty product is selected from a plurality of beauty products via a beauty product selection control 250. Beauty product selection may be driven by the user or may occur automatically or semi-automatically, such as by selecting a product complementary to some other information relating to the user and/or the user's activity. A list, for example, corresponding to the plurality of beauty products may be displayed, providing the subject with recommended and/or pre-selected options for simulation.

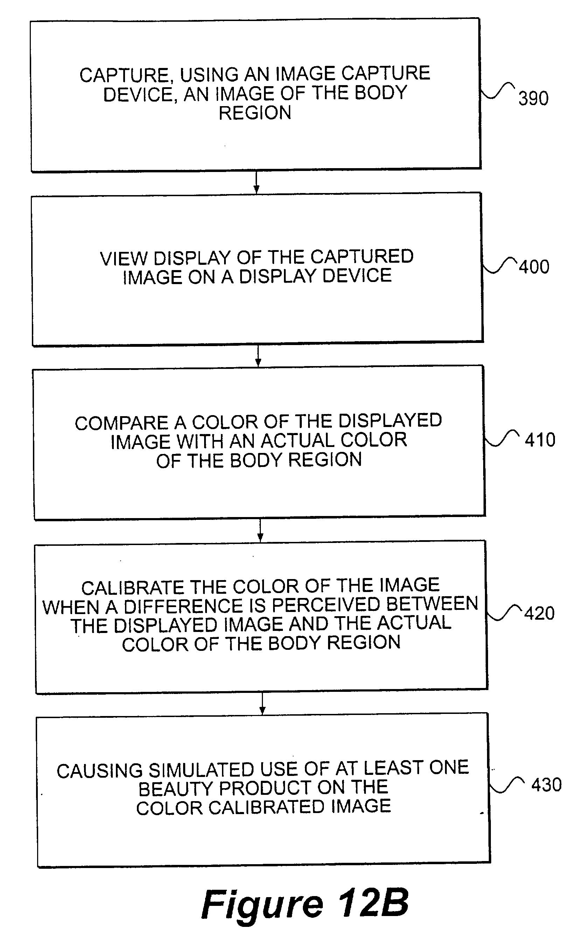

Figure 12B presents, from the user's perspective, the method of Fig. 12A. The method may involve capturing, using an image capture device, an image of the body region (390); viewing display of the captured image on a display device (400); comparing a color of the displayed image with an actual skin color of the body region (410); calibrating the color of the image when a difference is perceived between the displayed image and the actual color of the body region (420); and causing simulated use of at least one beauty product on the color-calibrated image (430).

Computer graphics techniques may be used to generate a multi-dimensional image and/or simulate the subject's external body condition. Such techniques may also be used to model the evolution of the external body condition over time. For example, a three dimensional or a two dimensional image of a human face may be defined by its edges or points. Next, those points may be linked together by lines to create a wire-frame rendering of the object representing the human face. In an exemplary embodiment, an MPEG-4 facial mesh characterized by Facial Definition Parameters (FDPs) may be used. Next, a two-dimensional image of the subject may be applied at the surface of the wire-frame. In some cases objects may be lit by a light source and may be shaded. Surfaces may be represented as polygons, or as B-spline patches, or by any other computer graphics technique. Any graphics application, such as OpenGL, Renderman, or VRML may be used for modeling an external body condition on a human anatomy.

A human face could be modeled with B-spline patches representing muscle patches on a representation of the human face. As part of representing facial muscles as B-spline patches, the nature and direction of muscle fibers may be taken into account. In general, the facial muscles are of two types: linear muscles and sphincter muscles. A linear muscle contracts toward an attachment on the bone such as the frontalis major muscle that raises the eyebrows. A sphincter muscle on the other hand, contacts around an imaginary central point such as the orbicularis oris muscle that draws them out together. In one exemplary embodiment, open B-spline patches may be used to simulate the linear muscles while closed B-spline may be used to simulate the sphincter muscles.

A human face may be modeled by noting that it is a layered structure composed of a skull, a muscle layer, an outer skin layer, and connecting tissue between the muscle layer and the outer skin layer. Tissue connecting the outer skin to muscles may be simulated with imaginary springs. Such a model is discussed in "A Plastic-Visco-Elastic Model for Wrinkles in Facial Animation and Skin Aging," by Wu et al. which is incorporated by reference in its entirety herein. Using this facial model in one exemplary embodiment, deformations associated with movements of face may be represented. Not only the elastic aspect of facial movement but also the plasticity of skin, which may develop with aging resulting in wrinkles, may also be incorporated as part of this facial model.

Using a modified version of the afore-mentioned model, in one exemplary embodiment, external body conditions, such as wrinkles may be simulated. An addition of a wrinkle may be used as an input to an existing mathematical model of the facial image, and the facial image may be modified accordingly. For example, a plasticity weighting factor associated with the part of the facial image where the wrinkle is to be added may be changed to cause simulation of the addition of the wrinkle. In one example, the mathematical model of the image may be modified when the subject submits a response to the self-evaluation prompt. In another example, a user may select a beauty product (for example, a wrinkle remover), and the mathematical model associated with the image may be modified to take into account the effect of the selected beauty product.

Other models and/or mathematical techniques may be used to simulate the user's self-evaluation and/or affects of beauty products. Optionally, these models and techniques may be used to simulate the affects of aging. In one example, rather than physically-based models, geometric models may be used to simulate an external body condition.

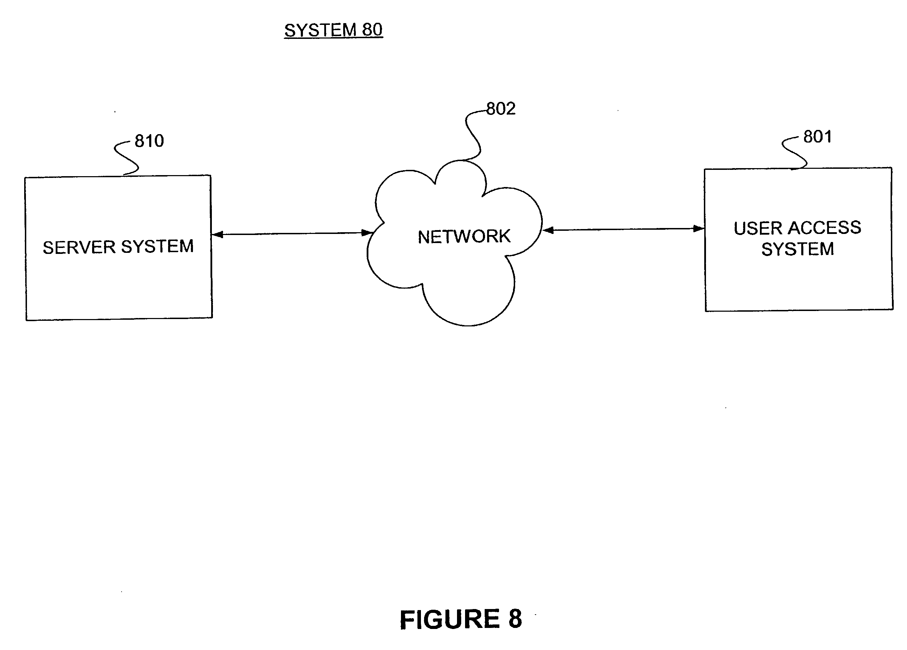

Figure 8 shows an example of a system 80 that may be used to practice at least portions of the methods consistent with principles of the present invention, but it should be understood that there are many alternative arrangements that are capable of being used. System 80 may include user access system 801 coupled, via network 802, to server system 810. For the sake of brevity, Figure 8 illustrates a single user access system coupled to a single server system. However, one skilled in the art will realize that system 80 may comprise any number of geographically dispersed user access systems coupled to a server system. Similarly, in alternative embodiments, the user access systems may be coupled to a collaborative network of central processors or server computers.

Network 802 may include a public network such as the Internet, a private network, a virtual private network, or any other mechanism for enabling communication between two or more nodes or locations. The network may include one or more of wired and wireless connections. User access system 801 and server system 810 may be, in an exemplary embodiment, operatively connected to network 802 by communication devices and software known in the art, such as are commonly employed by Internet service providers or as part of an Internet gateway.

In one embodiment, a subject may be able to capture a body image via image capture device 912 of Figure 9. Image capture device 912 may include a digital camera, digital video camera, scanner, web cam, or any other device capable of electronically capturing body images. For example, image capture device 912 may be camera 107 illustrated in the figures. When image capture device 912 is a scanner, the scanner could be used to obtain a digital image of a photograph obtained from a film camera, for example, or the scanner could be used to directly acquire the body image.

As illustrated in Figure 9, image capture device 912 may be operatively connected to user access system 801. User access system 801 may include, but is not limited to, a personal computer, mobile computing device (e.g., a PDA), mobile communications device (e.g., a cell phone), or any other structure that enables a user to remotely access information. In alternative embodiments, user access system 801 may be a kiosk or "dumb" terminal coupled to a central computer. For example, a beauty salon could have several kiosks linked to a central computer for customer use.

User access system 801 may include network interface 900, user interface 902, processor 904, display device 906, memory 908, and data port 910. Image capture device 912 may be coupled to access system 801 via data port 910 and may communicate with access system 801 using device driver 911 located in memory 908. As used herein, "memory" refers to any mechanism capable of storing information including, but not limited to, RAM, ROM, magnetic and optical storage, organic storage, audio disks, and video disks. Display device 906 may be configured to output the images captured by image capture device 912, as well as text and other information, by way of a cathode ray tube, liquid crystal, light-emitting diode, gas plasma, or any other type of display mechanism. Display device 906 may be, in one embodiment, computer monitor 104 illustrated in Figures 1, 3a, 3b, 4, 5, 6a, 6b, 7a, and 7b.

User interface 902 may include components such as keyboard 106 depicted in Figure 1. User interface 902 may include at least one button actuated by the user to input commands to select from a plurality of processor operating modes. User interface 902 may also be an input port connected by a wired, optical, or wireless connection for electromagnetic transmissions. In alternative embodiments, user interface 902 may include connections to other computer systems to receive the input commands and data therefrom. Moreover, user interface 902 may further include a mouse, a touch screen, and/or a data reading device such as a disk drive for receiving input data from and writing data to storage media such as magnetic and optical disks.

Processor 904 may be operatively configured to execute instructions received via memory 908, user interface 902, data port 910, and network interface 900. User access system 801 may be connected to network 802 via network interface 900, which may be operatively connected via a wired or wireless communications link. Network interface 900 may be a network interface card, unit, or any other type of dedicated network connection. In operation, network interface 900 may be used to send data to and receive data from network 802.

User access system 801 may also include audio port 915 coupled to audio input device 917 (e.g., a microphone) and an audio output device 919, such as a speaker. For clarity, Figure 9 illustrates audio input device 917 and audio output device 919 external to user access system 801; however, either or both of these devices may reside internal to the system.

As explained above, one or more mechanisms may be provided for capturing, storing, and altering images, as well as providing product recommendations. In one embodiment, these mechanisms may be software-based. The advice mechanism may include a neural network, decision tree, artificial intelligence engine, or any other logic-based apparatus or process.

For clarity of explanation, the functionality of the mechanisms described herein are distinguished. However, it is to be understood that, in operation, the functionality of these mechanisms may differ from what is described. For example, the mechanisms may be separate, each residing at different locations, or they may be integrated into one software package residing at a common location. Thus, the mechanism for operating the image capture device may be in the form of device driver 911 located in memory 908 of user access system 801, while the image processing and advice mechanisms may reside in software 1006 located in memory 908 of server system 810 (Figure 10).

In one embodiment, server system 810 may access software 1006 via network 802. However, as mentioned above, software 1006 could reside in other locations. For example, a CD-ROM or floppy disk containing software 1006 could be uploaded onto user access system 801. In another embodiment, user access system 801 could download the software from server 810 via network 802. In yet another embodiment, the software may be distributed and shared among server system 810 and user access system 801. Moreover, in other alternative embodiments, the aid of a third party may be involved. For example, the image processing may occur after transmission of the images over a network to an intervening server that uses highly specialized processes and routines for image manipulation and enhancement.

As shown in Figure 10, server system 810 may comprise components similar to those described in connection with user access system 801 including network interface 900 and processor 904. Further, database 1007 may be coupled to distributor server 810 for maintaining a plurality of images. Database 1007 may include a relational database, distributed database, object-oriented programming database, or any other aggregation of data that can be accessed, managed, and updated. While database 1007 is illustrated with a single icon, it is to be understood, as with all other components described herein, that its functionality may be distributed amongst several discrete components.

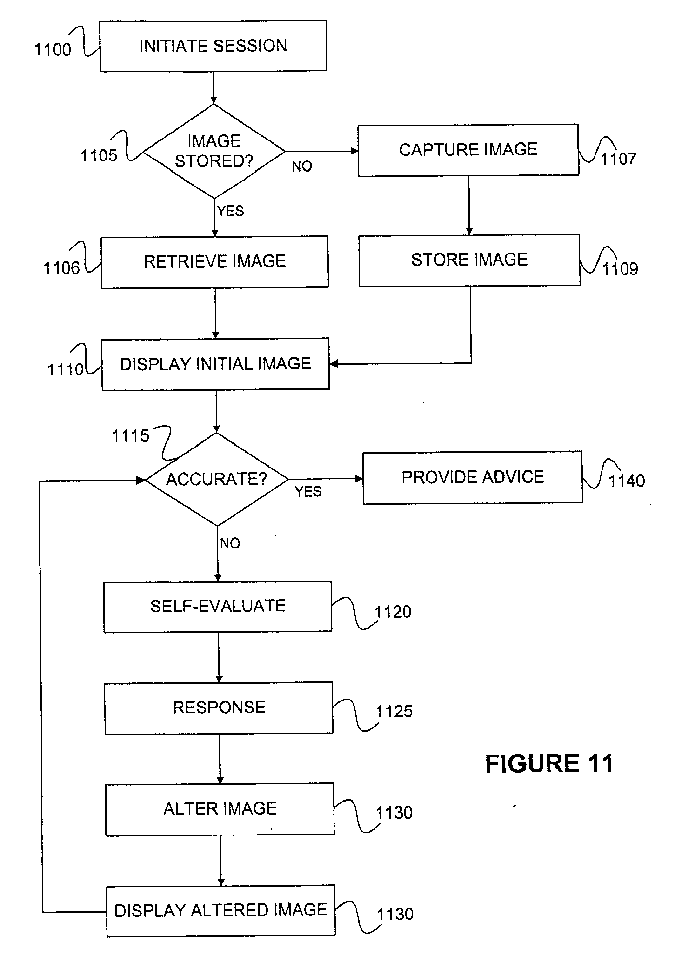

In operation, an exemplary embodiment of the present invention may function in accordance with the steps illustrated in the flowchart of Figure 11. However, it should be understood that other methods may be used to implement the invention, and even with the method disclosed in Figure 11, the particular order of events may vary without departing from the scope of the present invention. Further, certain steps may not be present and additional steps may be added without departing from the scope and spirit of the claimed invention.

As indicated in step 1100, a session may be initiated. In this exemplary embodiment, step 1100 may involve a subject communicating with server system 810 over network 802 via a website. This might further involve logging in with a username and/or password. Alternatively, this step may involve downloading and/or installing software on user access system 801, or simply running previously installed software. In yet another embodiment, step 1100 may involve inserting transportable storage media (e.g., a floppy disk), containing subject data, into a data reading device. This step may also involve installing or initiating image capture device 912.

Once a session is established, software 1006 may determine if the subject's image has been previously captured (step 1105). For example, this might involve checking the above-mentioned storage media for previously stored images. This may also involve searching memory 908 and/or database 1007. If it is determined that an image is not available, software 1006 will prompt the subject to capture an image. As indicted previously, prompting may involve displaying text and/or graphics on display device 906, and/or audibly prompting the subject via audio output device 919. As indicated in step 1107, a subject may capture an initial body image via image capture device 912. Upon capture, the image may be stored to memory, transportable storage media, or a database, as indicated by step 1109.

After capturing and storing the image, or retrieving a previously stored image (step 1106), the initial body image may be presented to the subject via display device 906 (step 1110). In one embodiment, software 1006 may contain a pre-determined sequence of prompts (e.g., queries) that will cause the subject to perform self-evaluations. For example, a subject may be prompted to evaluate her skin texture, followed by her skin color and then wrinkle intensity. As indicated in step 1115, a user may be prompted to indicate whether the captured image is accurate.

If it is determined that the subject is not satisfied with the image, or if software 1006 is running a pre-determined sequence of queries, the subject may be prompted to perform a self-evaluation (step 1120). For example, the subject may be asked to choose her skin tone from a plurality of selectable representations 20a, 20b, 20c, 20d, as illustrated in Figure 6a. Accordingly, the subject may respond by selecting the skin tone she feels most accurately reflects her actual skin, as indicated in step 1125. Once the subject responds to the prompt, the initial image may be altered based on the response (step 1130), to thereby reflect the self-evaluation. This alteration may be performed by software 1006.

As illustrated in step 1130, the altered image may then be displayed to the subject. After the altered image is presented, the subject may be asked whether the image is accurate (step 1115), and may make additional selections if she is not satisfied.

In an exemplary embodiment, the present invention may also provide beauty advice to the subject based on the image. This is illustrated in step 1140. Step 1140 may involve providing one or more of a product recommendation, remedial regimen, or preventative measure. This advice may be provided by software 1006.

It should be understood that processes described herein do not necessarily need to be practiced using any particular apparatus and may be implemented by any suitable combination of components. Further, various types of general purpose devices may be used in accordance with the teachings described herein. It may also prove advantageous to construct specialized apparatus to perform the method steps described herein.

Certain steps of the methods described herein are steps relating to "enabling" or "causing." It should be understood that that the activity for performing the "enabling," and/or "causing" could be either direct activity or indirect activity. For example, "enabling" and/or "causing" could relate to providing software to a user (e.g., via a network or via a storage media), providing one or more instructions and/or prompts (e.g., in electronic form and/or in hard copy form), providing network access through which a user-controlled device is enabled to act, providing a kiosk performing one or more activities, providing a dedicated device for performing one or more activities, transmitting computer readable data and/or software, and/or cooperating and/or partnering with an entity through whom the activity is performed.

It will be apparent to those skilled in the art that various modifications and variations can be made in the systems, methods and apparatus of the present invention and in the construction of this invention.

This application may discuss beauty products in connection with use by women. However, it is to be understood that such discussions are for exemplary purposes only. It is to be understood that the invention is equally applicable to all genders, and is not necessarily limited to the beauty industry. It is also to be understood that any functional aspect of the invention can be implemented via any location in the system or network, and data software may be resident at any location either in a network, at a stand-alone site, or on media in the custody and control of a user or subject.

It is to be further understood that the physical mechanisms (e.g. hardware, software, networks, systems) for implementing the methods of the invention are many. Networks, hardware and systems can be configured in a host of ways with software and hardware functionality residing at many alternative locations. In addition, systems other than the exemplary systems disclosed might be used to implement the invention. Therefore, it is to be understood that the methods of the invention are not limited to any particular structure.

Further, methods or portions thereof can be implemented in either an electronic environment, a physical environment, or combinations thereof. Thus, for example, although one or more portions of a method may occur in an electronic environment, a "purchase" portion of the method may occur in a brick and mortar store, or vice versa.

To the extent not inconsistent with the invention defined herein, the following global definitions are to be considered in interpreting the language of this patent and the claims herein. Where multiple definitions are provided, they should be considered as a single cumulative definition.

The term "image" may include one or more of two-dimensional and three-dimensional representations. In certain examples consistent with the invention, a plurality of images from different perspectives may be used to construct a three-dimensional image. In a broader sense, only a single image may be used. Depending on the embodiment, the term "image" may include either a visually perceptible image or electronic image data that may be either used to construct a visually perceptible image or to derive information about the subject. The image may be a body image corresponding to an anatomical portion of the subject, and may represent, for example, the subject's entire face, or a portion of the subject's face. The image may be a detailed picture (e.g., a digital image or a photograph) of a portion of the subject's body and/or a topological plot mapping contours of a portion of subject's body. If the image is representative of an external body condition, the image could be either an actual image showing the condition or an image including symbolizations of the condition, for example. The image may be an actual or a simulated image. Simulated images may include wholly or partially generated computer images, images based on existing images, and images based on stored features of a subject.

The term "image capture device", similar terms, and terms representing structures with similar functions may include one or more of a digital camera, webcam, film camera, analog camera, digital video camera, scanner, facsimile machine, copy machine, infrared imager, ultra-sound imaging device, or any other mechanism for acquiring an image of a subject's external body condition, an image of the subject's countenance, an/or an image of the subject's skin. An ultrasonic device might provide skin thickness information, or it might create a map on an area of the external location. Thus, the term "image" as used herein may be broader than a picture. Combinations of image capture devices may be used. For example, an image captured on photographic paper using a film camera might then be scanned on a flat bed scanner to create another image.

The term "capturing (an image)", or any form thereof, refers to the use of an image capture device to acquire an image. "Capturing" may refer to the direct act of using the image capture device to acquire the image. It may also include indirect acts to promote acquisition. To this end, "capturing" may include the indirect acts of providing access to hardware, or to at least one of a client-based algorithm and a server-based algorithm for causing the image capture device to capture an image. This may be accomplished by providing a user with software to aid in the image capture process, or providing the user with access to a network location at which the software resides. Also consistent with certain embodiments of the invention, capturing may include at least one of receiving an instruction from the subject to capture an image, indicating to the subject before the image is captured, and indicating to the subject when the image is captured.

The term "image processing technique" or similar terms, may include a software program, computer, application specific integrated circuit, electronic device and/or a processor designed to identify in an image one or more characteristics, such as a skin condition. Such techniques may involve binarization, image partitioning, Fourier transforms, fast Fourier transforms (FFTs), and/or discrete cosine transforms may be performed on all or part of the image, resulting in coefficients. Based on the coefficients, conditions may be located, as known in the art. Artificial intelligence, such as fuzzy logic, neural networks, genetic programming and decision tree programming, may also be used to identify conditions. Alternatively, one or more digital filters may be passed through the image for locating specific conditions. These examples are provided for illustrative purposes with the understanding that any image processing technique may be used.

The term "network interface" or similar terms, refer to any mechanism for aiding communications between various nodes or locations in a network. A network interface may include, for example a bus, a modem, or any other input/output structure. A network interface may permit a connection to any network capable of being connected to an input and/or output module located within at least one or more of the following exemplary networks: an Ethernet network, an Internet Protocol network, a telephone network, a radio network, a cellular network, or any mechanism for permitting communication between two or more modes or remote locations. In some invention embodiments, a network interface might also included a user interface.

The term "user interface" may include at least one component such as a keyboard, key pad, mouse, track ball, telephone, scanner, microphone, touch screen, web cam, interactive voice response system (IVR), voice recognition system or any other suitable input mechanism for conveying information. A user interface may also include an input port connected by a wired, optical, or wireless connection for electromagnetic transmissions. In some embodiments, a user interface may include connections to other computer systems to receive the input commands and data therefrom. User interface may further include a data reading device such as a disk drive for receiving input data from and writing data to storage media such as magnetic and optical disks.

As used herein terms such as "external body condition", "skin condition", and "actual condition" refer to conditions of at least one of the skin, teeth, hair, eyebrows, eyelashes, body hair, facial hair, fingernails, and/or toenails, or any other externality. Examples of skin conditions may include elasticity, dryness, cellulitis, sweating, aging, wrinkles, exfoliation, desquamation, homogeneity of color, creases, liver spots, clarity, lines, micro-circulation, shininess, softness, smoothness, tone, texture, matitty, hydration, sag, suppleness, stress, springiness, firmness, sebum production, cleanliness, translucency, luminosity, irritation, redness, vasocolation, vasomotion, vasodilation, vasoconstriction, pigmentation, freckles, blemishes, oiliness, pore distribution, pore size, moles, birthmarks, acne, blackheads, whiteheads, pockmarks, warts, pustules, boils, blisters, marks, smudges, specks, and other characteristics associated with the subject's skin. Examples of hair conditions may include keratin plug, length, dryness, oiliness, dandruff, pigmentation, thickness, density, root conditions, split ends, hair loss, hair thinning, scales, staging, cleanliness and other properties related to the subject's hair. Examples of fingernail and toenail conditions may include split nails, delaminating, brilliancy, lines, spots, coloration, gloss, strength, brittleness, thickness, hangnail, length, and other characteristics related to the subject's nails. Other conditions may include, for example, size and proportion of facial features, teeth discoloration, and any other aesthetic-related conditions of the user.

"Enabling", "facilitating", and "causing" an action refer to one or more of a direct act of performing the action, and any indirect act of encouraging or being an accessory to the action. Thus, the terms include partnering or cooperating with an entity who performs the action and/or referring commerce to or having commerce referred from an entity who performs the action. Other examples of indirect activity encompassed within the definitions of "enabling", "facilitating", and "causing" may include providing a subject with one or more of tools to knowingly aid in performing the action, providing instructions on how to perform the action, providing prompts or cues to perform the action, or expressly encouraging performance of the action. Indirect activity may also include cooperating with an entity who either directly performs the action or who helps another perform the action. Tools may include software, hardware, or access (either directly, through hyperlink, or some other type of cooperation or partnering) to a network location (e.g., web site) providing tools to aid in performing the action. Thus, phrases such as "enabling access" and "enabling display" do not necessary require that the actor actually access or display anything. For example, the actor may perform the enabling function by affiliating with an entity who performs the action, or by providing instructions, tools, or encouragement for another to do the accessing and displaying.

Forms of the word "displaying" and like terms may also include indirect acts such as providing content for transmission over a network to a display device, regardless of whether the display device is in the custody or control of the sender. Any entity in a chain of delivering information for display performs an act of "displaying", as the term is used herein.

Likewise, the term "providing" includes direct and indirect activities. For example, providing access to a computer program may include at least one of providing access over a network to the computer program, and creating or distributing to the subject a computer program configured to run on the subject's workstation or computer. For example, a first party may direct network traffic to (either through electronic links or through encouragement to visit) a server or web site run by a second party. If the second party maintains a particular piece of software thereon, then it is to be understood that within the meaning of "providing access" as used herein, the first party is said to provide access to the particular software. Or if the first party directs a subject to a second party who in turn ships the particular software to the user, the first party is said to provide the user with access to the particular software. (Of course, in both of the above instances, the second party would also be providing access within the meaning of the phrase as used herein.)

"Receiving" may include at least one of acquisition via a network, via verbally communication, via electronic transmission, via telephone transmission, in hard-copy form, or through any other mechanism enabling reception. In addition, "receiving" may occur either directly or indirectly. For example, receipt may occur through a third party acting on another party's behalf, as an agent of another, or in concert with another. Regardless, all such indirect and direct actions are intended to be covered by the term "receiving" as used herein. A received request, for example, may take one of many forms. It may simply be a checked box, clicked button, submitted form or oral affirmation. Or it might be a typed or handwritten textual request. Receiving may occur through an on-line interest form, e-mail, facsimile, telephone, interactive voice response system, or file transfer protocol transmitted electronically over a network at a web site, an internet protocol address, or a network account. A request may be received from a subject for whom information is sought, or an entity acting on the subject's behalf. "Receiving" may involve receipt directly or indirectly through one or more networks and/or storage mediums. Receipt may occur physically such as in hard copy form, via mail delivery or other courier delivery.

Forms of the word "maintain" are used broadly to include gathering, storing, accessing, providing access to, or making something available for access, either directly or indirectly. For example, those who maintain information include entities who provide a link to a site of a third party where the information is stored.

Consistent with the concepts set forth above, all other recited actions such as, for example, obtaining, determining, generating, selecting, applying, simulating, presenting, etc, are inclusive of direct and indirect actions. Thus, for purposes of interpreting the following claims, an entity performs a recited action through either direct or indirect activity. Further examples of indirect activity include sending signals, providing software, providing instructions, cooperating with an entity to have the entity perform the action, outsourcing direct or indirect actions, or serving in any way as an accessory to the specified action.

The term "product" is used to generically refer to tangible merchandise, goods, services, and actions performed. A "beauty product," "beauty care product," "cosmetic product" or similar terms, refer to products (as defined above) for effecting one or more external body conditions, such as conditions of the skin, hair and nails. Examples of tangible merchandise forms of beauty products include cosmetic goods, such as treatment products, personal cleansing products, and makeup products, in any form (e.g., ointments, creams, gels, sprays, supplement, ingesta, inhalants, lotions, cakes, liquids, and powders.)

Examples of services forms of beauty products include hair styling, hair cutting, hair coloring, hair removal, skin treatment, make-up application, and any other offering for aesthetic enhancement. Examples of other actions performed include massages, facial rubs, deep cleansings, applications of beauty product, exercise, therapy, or any other action effecting the external body condition whether performed by a professional, the subject, or an acquaintance of the subject.

The following is exemplary and non-exhaustive listing of a few beauty products- scrubs, rinses, washes, moisturizers, wrinkle removers, exfoliates, toners, cleansers, conditioners, shampoos, cuticle creams, oils, and anti-fungal substances, anti-aging products, anti-wrinkle products, anti-freckle products, skin conditioners, skin toners, skin coloring agents, tanners, bronzers, skin lighteners, hair coloring, hair cleansing, hair styling, elasticity enhancing products, agents, blushes, mascaras, eyeliners, lip liners, lipsticks, lip glosses, eyebrow liners, eye shadows, nail polishes, foundations, concealers, dental whitening products, cellulite reduction products, hair straighteners and curlers, and weight reduction products. A beauty care treatment regimen may involve the administration of one or more products, as defined above.

The terms "beauty advice", "beauty guidance", and similar terms are used interchangeably to refer to the provision of beauty related information to a subject. Advice or guidance includes one or more of beauty product recommendations (e.g., cosmetic product recommendations for products to treat conditions the subject is prompted to evaluate), remedial measures, preventative measures, predictions, prognoses, price and availability information, application and use information, suggestions for complementary products, lifestyle or dietary recommendations, or any other information intended to aid a subject in a course of future conduct, to aid a subject in understanding past occurrences, to reflect information about some future occurrences related to the subject's beauty or to aid a subject in understanding beauty products, as defined above.

The terms "beauty product" and "cosmetic product" refer to a product as defined in the Council Directive 93/35/EEC of 14 June 1993

The term "network" may include a public network such as the Internet or a telephony network, a private network, a virtual private network, or any other mechanism for enabling communication between two or more nodes or locations. The network may include one or more of wired and wireless connections. Wireless communications may include radio transmission via the airwaves, however, those of ordinary skill in the art will appreciate that various other communication techniques can be used to provide wireless transmission including infrared line of sight, cellular, microwave, satellite, blue-tooth packet radio and spread spectrum radio. Wireless data may include, but is not limited to, paging, text messaging, e-mail, Internet access and other specialized data applications specifically excluding or including voice transmission.

In some instances consistent with the invention, a network may include a courier network (e.g. postal service, United Parcel Service, Federal Express, etc.). Other types of networks that are to be considered within the scope of the invention include local area networks, metropolitan area networks, wide area networks, ad hoc networks, or any mechanism for facilitating communication between two nodes or remote locations.

"Artificial intelligence" (Al) is used herein to broadly describe any computationally intelligent systems that combine knowledge, techniques, and methodologies. An Al engine may be any system configured to apply knowledge and that can adapt itself and learn to do better in changing environments. Thus, the Al engine may employ any one or combination of the following computational techniques: neural network, constraint program, fuzzy logic, classification, conventional artificial intelligence, symbolic manipulation, fuzzy set theory, evolutionary computation, cybernetics, data mining, approximate reasoning, derivative-free optimization, decision trees, or soft computing. Employing any computationally intelligent techniques, the Al engine may learn to adapt to unknown or changing environment for better performance. Al engines may be implemented or provided with a wide variety of components or systems, including one or more of the following: central processing units, co-processors, memories, registers, or other data processing devices and subsystems.

Al engines may be trained based on input such as product information, expert advice, user profile, or data based on sensory perceptions. Using input an Al engine may implement an iterative training process. Training may be based on a wide variety of learning rules or training algorithms. For example, the learning rules may include one or more of the following: back-propagation, real-time recurrent learning, pattern-by-pattern learning, supervised learning, interpolation, weighted sum, reinforced learning, temporal difference learning, unsupervised learning, or recording learning. As a result of the training, Al engine may learn to modify its behavior in response to its environment, and obtain knowledge. Knowledge may represent any information upon which Al engine may determine an appropriate response to new data or situations. Knowledge may represent, for example, relationship information between two or more products. Knowledge may be stored in any form at any convenient location, such as a database.

Since Al engine may learn to modify its behavior, information describing relationships for a universe of all combinations of products may not need to be maintained by the Al engine or any other component of the system.

"Personal information", "subject specific information", "user specific information", "user profile", "personal characteristics", "personal attributes", "profile information", and like terms (collectively referred to in this section as "personal information") may broadly encompass any information about the subject or user. Such information may, for example, fall within categories such as physical characteristics, fashion preferences, demographics, nutritional information, cosmetic usage information, medical history information, environmental information, beauty product usage information, lifestyle, and may include information such as name; age; birth date; height; weight; ethnicity; eating habits; vacation patterns; geographic location of the individual's residence, location, or work; work habits; sleep habits; toiletries used; exercise habits; relaxation habits; beauty care habits; smoking and drinking habits; sun exposure habits; use of sunscreen; propensity to tan; number of sunburns and serious sunburns; dietary restrictions; dietary supplements or vitamins used; diagnosed conditions affecting the external body, such as melanoma; an image, such as a picture or a multimedia file of the subject; facial feature characteristics; family history information such as physical characteristics information about relatives of the subject (e.g., premature balding, graying, wrinkles, etc.); external body condition (as defined previously); color preferences, clothing style preferences, travel habits; entertainment preferences; fitness information; adverse reactions to products, compounds, or elements (e.g., sun exposure); body chemistry, use of prior beauty care products and their effectiveness; purchasing, shopping, and browsing habits; hobbies; marital status; whether the subject is a parent; country of residence; region of residence; birth country and region; religious affiliation; political affiliation; whether the subject is an urban dweller suburban dweller or rural area dweller; size of urban area in which the subject lives; whether the subject is retired; annual income, sexual preference, or any other information reflecting habits, preferences, or affiliations of the subject.

Personal information may also include information electronically gleaned by tracking the subject's electronic browsing or purchasing habits, or as the result of cookies maintained on the subject's computer, responses to surveys, or any other mechanism providing information related to the subject. In addition, personal information may be gathered through non-electronic mechanisms such as hard copy surveys, personal interviews, or consumer preference polls.

"Complementary" and "complementary product" refers to one or more of physical, physiological, biologically, and aesthetic compatibility. A product may be complementary with one or more of another product, a group of products, or a subject. In that latter instance, whether a product is considered "complementary" may be a function of personal information of the subject. Thus, for example a product may be complementary if it is unlikely to cause an adverse allergic reaction; if it physically blends well with another product; or if it is aesthetically consistent with the subject or one or more other products. Aesthetic compatibly may refer to the fact that two products are aesthetically appealing (or do not clash) when worn together. The identification of a complementary product may also be based on product characteristics, user preferences, survey data, or expert advice.

As used herein, the words "may" and "may be" are to be interpreted in an open-ended, non-restrictive manner. At minimum, "may" and "may be" are to be interpreted as definitively including structure or acts recited. Further, the word "or" is to be interpreted in the conjunctive and the disjunctive.

While flow charts presented herein illustrate a series of sequential blocks for exemplary purposes, the order of blocks is not critical to the invention in its broadest sense. Further, blocks may be omitted and others added without departing from the spirit of the invention. Also, the invention may include combinations of features described in connection with differing embodiments.

Although a focus of the disclosure may be on server-side methods, it is nevertheless to be understood that the invention includes corresponding client-side methods, software, articles of manufacture, and computer readable media, and that computer readable media can be used to store instructions for some or all of the methods described herein. Further, it is to be understood that disclosed structures define means for implementing the functionality described herein, and that the invention includes such means for performing the disclosed functions.

In the foregoing Description of Exemplary Embodiments, various features are grouped together in a single embodiment for purposes of streamlining the disclosure. This method of disclosure is not to be interpreted as reflecting an intention that the claimed invention requires more features than are expressly recited in each claim. Rather, as the following claims reflect, inventive aspects lie in less than all features of a single foregoing disclosed embodiment. Thus, the following claims are hereby incorporated into this Description of the Exemplary Embodiments, with each claim standing on its own as a separate embodiment of the invention.

## Claims

1. A method of constructing a body image, the method comprising:
prompting a subject to capture, using an image capture device, at least one initial body image of the subject;
causing presentation of at least one prompt prompting the subject to self-evaluate an actual condition of the subject's own body;
enabling the subject to respond to the at least one prompt; and
enabling the initial body image to be altered based on the subject's response to the at least one prompt, to thereby reflect in the altered image the self-evaluation of the subject.

2. Method according to the preceding claim, wherein the initial image is present in two-dimensional form.

3. Method according to claim 1, wherein the initial image is presented in three-dimensional form.

4. Method according to any one of the preceding claims, wherein the method comprises at least one of transmitting information via a network and receiving information via the network.

5. Method according to any one of the preceding claims, wherein the image capture device comprises at least one of a digital camera, a scanner, a web cam, a camcorder, and a film camera.

6. Method according to the preceding claim, wherein prompting the subject includes instructing the subject to use the image capture device.

7. Method according to any one of the preceding claims, wherein the at least one prompt comprises at least one question.

8. Method according to any one of the preceding claims, wherein the at least one prompt is presented graphically in multiple choice form.

9. Method according to any one of the preceding claims, wherein the at least one prompt prompts the subject to self-evaluate at least one of skin pigmentation, skin texture, skin sheen, skin tone, skin mattiness, skin lines, skin wrinkles, distribution of wrinkles, intensity of wrinkles, intensity of pores, depth of pores, color tone, color homogeneity, spots, freckles, shininess, oiliness, roughness, distribution of hair, thickness of hair, length of hair, density of hair, on the subject's own body.

10. Method according to any one of the preceding claims, wherein the at least one prompt prompts the subject to self-evaluate an actual skin condition of the subject's own body chosen among at least one of skin pigmentation, skin texture, skin sheen, skin tone, skin mattiness, skin lines, skin wrinkles, distribution of wrinkles, intensity of wrinkles, intensity of pores, depth of pores, color tone, color homogeneity, spots, freckles, shininess, oiliness, and roughness.

11. Method according to any one of the preceding claims, wherein the body image includes a facial image, and wherein the at least one prompt prompts the subject to self-evaluate characteristics of the subject's own face.

12. Method according to any one of the preceding claims, further comprising enabling a subject to view a time-lapse projection of the altered image.

13. Method according to the preceding claim, wherein the time-lapse projection is based on an assumption that the subject has followed a particular beauty regimen.

14. Method according to claim 12, wherein the time-lapse projection is based on an assumption that the subject has not followed a particular beauty regimen.

15. Method according to any one of the preceding claims, wherein the initial body image is a facial image, wherein the actual condition comprises at least one of skin pigmentation, skin texture, skin sheen, skin tone, skin mattiness, skin wrinkles, and skin lines, wherein the actual condition is located in regions of at least one of the eyes, forehead, cheeks, lips, brow, and nose, and wherein the image is altered to reflect the subject's self-evaluation of the actual condition.

16. Method according to any one of the preceding claims, further comprising causing at least a portion of the initial body image to be displayed on a display device, causing at least one control element to be displayed on the display device, and enabling activation of the control element to alter said at least a portion of the image displayed on the display device.

17. Method according to the preceding claim, wherein the actual condition is wrinkles and wherein movement of the control element in a first direction causes an increase in the appearance of wrinkles on said at least a portion of the image displayed on the display device and movement of the control element in a second direction causes a decrease in the appearance of wrinkles on said at least a portion of the image displayed on the display device.

18. Method according to any one of the two preceding claims, wherein the at least one control element comprises first and second control elements and the actual condition is wrinkles, wherein activation of the first control element causes an increase in the appearance of wrinkles on said at least a portion of the image displayed on the display device and activation of the second control element causes a decrease in the appearance of wrinkles on said at least a portion of the image displayed on the display device.

19. Method according to any one of the preceding claims, further comprising causing a plurality of selectable condition representations to be displayed on the display device, and enabling the subject to select at least one of the condition representations.

20. Method according to the preceding claim, wherein the at least one prompt is associated with the plurality of selectable condition representations and wherein the subject's response to the prompt comprises selection of at least one of the representations.

21. Method according to the preceding claim, wherein enabling the initial body image to be altered comprises enabling the initial body image to be altered to include at least one condition representation selected by the subject.

22. Method according to any one of claims 19 to 21, wherein the plurality of selectable condition representations are caused to be displayed in at least one of a one-by-one fashion and a simultaneous fashion.

23. Method according to the preceding claim, wherein the plurality of selectable condition representations are displayed by means of one of a slide show presentation and a movie presentation.

24. Method according to any one of claims 20 to 23, wherein the actual condition comprises wrinkles and wherein the selectable condition representations are representation of varying appearances of wrinkles.

25. Method according to any one of the preceding claims, further comprising causing display on a display device of at least a portion of the initial body image, and wherein the at least one prompt further prompts the subject to compare said at least a portion of the body image displayed on the display device to the subject's own body.

26. Method according to the preceding claim, wherein the enabling of the subject to respond to the at least one prompt comprises enabling the subject to indicate whether said at least a portion of the body image displayed on the display device accurately represents the actual condition of the subject's own body, and wherein enabling the initial image to be altered comprises enabling the image to be altered when the subject believes said at least a portion of the body image displayed on the display device does not accurately represent the actual condition.

27. Method according to any one of the preceding claims, wherein the initial body image is an image of at least a portion of the subject's face.

28. Method according to any one of the preceding claims, further comprising enabling the subject to store the altered image.

29. Method according to any one of the preceding claims, further comprising enabling the subject to receive information about at least one beauty product for treating the actual condition.

30. Method according to the preceding claim, wherein the information comprises a recommendation to use the at least one beauty product.

31. Method according to any one of the two preceding claims, wherein enabling the subject to receive information comprises enabling the subject to view a time lapse projection of the altered image based on an assumption of the subject using the at least one beauty product to treat the actual condition.

32. Method according to any one of claims 29 to 31, wherein the at least one beauty product is a cosmetic product.

33. Method according to any one of the preceding claims, further comprising enabling the subject to simulate use of at least one beauty product on the altered image.

34. Method according to the preceding claim, further comprising enabling the subject to select the at least one beauty product from a plurality of beauty products.

35. Method according to any one of the two preceding claims, wherein the at least one beauty product comprises at least one makeup product.

36. Method according to any one of claims 33 to 35, wherein the at least one beauty product comprises at least one product for treating the actual condition.

37. Method according to any one of the preceding claims, wherein the method further comprises enabling extraction of at least one portion of the initial body image and enabling the subject to view the extracted portion on a display device, and wherein the at least one prompt prompts the subject to self-evaluate an actual condition of an actual portion of the subject corresponding to the extracted portion.

38. Method according to the preceding claim, wherein the enabling the initial body image to be altered comprises altering at least the extracted portion of the initial body image based on the subject's response to the prompt.

39. Method according to any one of the two preceding claims, wherein the enabling extraction comprises enabling extraction of a plurality of portions of the initial body image.

40. Method according to any one of the preceding claims, further comprising causing display, on a display device, of a representation of the initial body image having fuzzy distortion.

41. Method according to the preceding claim, wherein the displayed image is altered, based on the subject's response to the at least one prompt, to remove at least a portion of the fuzzy distortion.

42. Method according to the preceding claim, wherein each response to the at least one prompt causes further removal of the fuzzy distortion.

43. Method according to any one of the two preceding claims, wherein the displayed image is altered so that a representation of the actual condition is substituted in place of said at least a portion of the fuzzy distortion.

44. Method according to any one of the preceding claims, enabling construction of an image of an external body condition, wherein the at least one initial body image of the subject is at least one representative image of an external body condition of the subject, wherein said at least one prompt is a first prompt, wherein the altered image is an enhanced image generated based on the representative image and the response to the at least one first prompt, and intended to more accurately portray the subject's external body condition, and wherein the method further comprises:
causing the enhanced image to be displayed to the subject;
causing presentation to the subject of at least one second prompt prompting the subject to indicate whether the enhanced image is accurate; and
enabling the subject to respond to the at least one second prompt.

45. Method according to the preceding claim, wherein the at least one second prompt comprises at least one question.

46. An electronic system for constructing an image of an external body condition, the system comprising:
a processor for receiving at least one representative image of an external body condition of a subject;
a first prompt generating module for presenting to the subject at least one first prompt prompting the subject to self-evaluate at least one of color and texture of the external body condition;
a first input receiving module for receiving from the subject at least one response to the at least one first prompt;
an image generator, for generating from the actual image and the at least one response, an enhanced image intended to more accurately portray the subject's external body condition;
a image module for causing the enhanced image to be displayed to the subject;
a second prompt generating module for causing at least one second prompt to be presented to the subject, the second prompt prompting the subject to confirm an accuracy of the enhanced image; and
a second input receiving module for receiving from the subject a response to the at least one second prompt.

47. A method of constructing a body image, the method comprising:
receiving an initial body image of a subject, wherein the initial body image is an image captured using an image capture device;
presenting at least one prompt prompting the subject to self-evaluate an actual condition of the subject's own body;
receiving the subject's response to the at least one prompt; and
enabling the initial body image to be altered based on the subject's response to the at least one prompt, to thereby reflect in the altered image the self-evaluation of the subject.

48. A method of constructing an image of an external body condition, the method comprising:
prompting a subject to capture, using an image capture device, at least one representative image of an external body condition of a subject;
causing presentation to the subject of at least one first prompt prompting the subject to self-evaluate at least one of actual color and actual texture of the external body condition;
enabling the subject to respond to the at least one first prompt;
enabling generation, based on the representative image and the at least one response, of an enhanced image intended to more accurately portray the subject's external body condition;
causing the enhanced image to be displayed to the subject;
causing presentation to the subject of at least one second prompt prompting the subject to indicate whether the enhanced image is accurate; and
enabling the subject the subject to respond to the at least one second prompt.

49. A method of constructing an image of an external body condition, the method comprising:
receiving at least one representative image of an external body condition of a subject;
causing presentation to the subject of at least one first prompt prompting the subject to self-evaluate at least one of actual color and actual texture of the external body condition;
receiving from the subject at least one response to the at least one first prompt;
generating, based on the representative image and the at least one response, an enhanced image intended to more accurately portray the subject's external body condition;
causing the enhanced image to be displayed to the subject;
causing presentation to the subject of at least one second prompt prompting the subject to indicate whether the enhanced image is accurate; and
receiving from the subject at least one response to the at least one second prompt.

50. A method of constructing a facial image, the method comprising:
prompting a subject to cause input into a computer of a facial image of the subject, wherein the facial image includes at least one bias element that may tend to cause the subject to perceive that the facial image may inaccurately portray reality;
enabling an identification in the facial image of the at least one bias element;
enabling the subject to participate in selecting a new visual element to replace the at least one identified bias element;
enabling the construction of an altered image from the facial image by replacing the at least identified one bias element with the new visual element; and
enabling the altered image to be displayed to the subject.

51. A method of constructing a body image, the method comprising:
causing presentation of at least one initial body image of the subject;
causing presentation of at least one prompt prompting the subject to self-evaluate an actual condition of the subject's own body;
enabling the subject to respond to the at least one prompt; and
enabling the initial body image to be altered based on the subject's response to the at least one prompt, to thereby reflect in the altered image the self-evaluation of the subject.

52. A method of enabling color-calibrating of a self-image for use in simulating a beauty product use, the method comprising:
prompting a subject to capture, using an image capture device, an image of a body region of the subject;
enabling the display of the captured image to the subject on a display device;
prompting the subject to compare a color of the displayed image with an actual color of the subject;
enabling the subject to calibrate the color of the image when the subject perceives a difference between the displayed image and the actual skin color; and
enabling the subject to simulate use of at least one beauty product on the color calibrated image.

53. A method of color-calibrating an image for use in simulating a beauty product use, the method comprising:
capturing, using an image capture device, an image of a body region;
viewing display of the captured image on a display device;
comparing a color of the displayed image with an actual color of the body region;
calibrating the color of the image when a difference is perceived between the displayed image and the actual color of the body region; and
causing a simulation of use of at least one beauty product on the color calibrated image.
